(19) Europäisches Patentamt / European Patent Office / Office européen des brevets

(11) **EP 4 393 397 B1**

(12) **EUROPEAN PATENT SPECIFICATION**

(45) Date of publication and mention of the grant of the patent:
**05.08.2026 Bulletin 2026/32**

(21) Application number: **22383301.3**

(22) Date of filing: **28.12.2022**

(51) International Patent Classification (IPC):
**A61B 5/16** (2006.01)   **A61B 5/00** (2006.01)

(52) Cooperative Patent Classification (CPC):
**A61B 5/168; A61B 5/726; A61B 5/7267;**
A61B 5/0075; A61B 5/055; A61B 5/374; A61B 5/377

(54) **COMPUTER IMPLEMENTED METHOD FOR SELECTING FUNCTIONAL BIOMARKERS TO IDENTIFY A TARGET CONDITION IN A SUBJECT**

COMPUTERIMPLEMENTIERTES VERFAHREN ZUR AUSWAHL FUNKTIONELLER BIOMARKER ZUR IDENTIFIZIERUNG EINES ZIELZUSTANDS IN EINER PERSON

PROCÉDÉ MIS EN UVRE PAR ORDINATEUR POUR SÉLECTIONNER DES BIOMARQUEURS FONCTIONNELS POUR IDENTIFIER UN ÉTAT CIBLE CHEZ UN SUJET

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**03.07.2024 Bulletin 2024/27**

(73) Proprietor: **Newmanbrain, S.L.**
**03202 Elche (ES)**

(72) Inventors:
• **IBAÑEZ BALLESTEROS, Joaquin**
**03540 ALICANTE (ES)**
• **MOLINA RODRIGUEZ, Sergio**
**03181 TORREVIEJA (ES)**
• **BELMONTE MARTINEZ, Carlos**
**03550 SAN JUAN DE ALICANTE (ES)**

(74) Representative: **Sugrañes, S.L.P.**
**Calle Provenza 304**
**08008 Barcelona (ES)**

(56) References cited:
• **GHORBANIAN P. ET AL: "Continuous Wavelet Transform EEG Features of Alzheimer's Disease", ASME 2012 5TH ANNUAL DYNAMIC SYSTEMS AND CONTROL CONFERENCE JOINT WITH THE JSME 2012 11TH MOTION AND VIBRATION CONFERENCE, 13 January 2015 (2015-01-13), pages 567 - 572, XP93052466, ISBN: 978-0-7918-4529-5, Retrieved from the Internet <URL:https://www.researchgate.net/publication/267491844> DOI: 10.1115/DSCC2012-MOVIC2012-8684**
• **DANIEL A ABRAMS ET AL: "Inter-subject synchronization of brain responses during natural music listening", EUROPEAN JOURNAL OF NEUROSCIENCE, OXFORD UNIVERSITY PRESS, GB, vol. 37, no. 9, 11 April 2013 (2013-04-11), pages 1458 - 1469, XP071869157, ISSN: 0953-816X, DOI: 10.1111/EJN.12173**
• **MAXWELL A BERTOLERO ET AL: "Learning differentially reorganizes brain activity and connectivity", ARXIV.ORG, CORNELL UNIVERSITY LIBRARY, 201 OLIN LIBRARY CORNELL UNIVERSITY ITHACA, NY 14853, 20 October 2018 (2018-10-20), XP081759367**

EP 4 393 397 B1

- ORTUÑO-MIRÓ SERGIO ET AL: "Identifying ADHD boys by very-low frequency prefrontal fNIRS fluctuations during a rhythmic mental arithmetic task", JOURNAL OF NEURAL ENGINEERING, vol. 20, no. 3, 23 May 2023 (2023-05-23), Bristol, GB, XP093052559, ISSN: 1741-2560, Retrieved from the Internet <URL:https://iopscience.iop.org/article/10.1088/1741-2552/acad2b/pdf> DOI: 10.1088/1741-2552/acad2b

**Description**

Technical field of the invention

The invention relates to a computer implemented method for selecting functional biomarkers to identify a target condition in a subject, such as attentiondeficit/hyperactivity disorder (ADHD). The invention also relates to a classifier for identifying the target condition using the selected biomarkers and to a computer implemented method for identifying the target condition using the trained classifier.

Background of the invention

Attention-deficit/hyperactivity disorder (ADHD) is recognized as a highly prevalent neurodevelopmental disorder in school-age children worldwide, often persisting into adolescence and adulthood, and frequently overlapped with other psychiatric comorbidities. It is currently accepted that ADHD is a complex, heterogeneous disorder, in which different expressions of impairment along with variable trajectories must be recognized in order to adopt personalized approaches that best target an individual. This is of crucial importance because, even despite serious distress/impairments, many patients lead rewarding and productive lives when properly managed.

Diagnosis of ADHD is event today based mainly on clinical signs and symptoms that require a detailed evaluation by an expert clinician through interviews with parents/caregivers and/or the patient himself, if applicable. Noteworthy, diagnosis cannot be solely based on rating scales, neuropsychological test or brain imaging. Despite the criticisms that argue a risk of subjectivity, the current consensus supports the validity of the diagnostic criteria applied by well-trained professionals. However, even for a specialist, clinical evaluation is quite time-consuming and requires several visits to be thoroughly performed. Besides, the significant shortage of trained professionals also contributes to a frequent delay in diagnosis or even to overlook some cases. From a developmental perspective, an early diagnosis is very likely to be of value for more effective pharmacological and psychosocial interventions. In this view, there is a need for objective biomarkers as useful adjunctive indicators to alleviate the workload of diagnoses and treatment follow-up.

Numerous studies have tried to assess ADHD through different objective diagnostic tools, most using functional or structural MRI and EEG, with other modalities (MEG, EKG, etc.) being deployed less frequently, and with an increasing use of artificial intelligence (AI) techniques.

Noticeable efforts in MRI and fMRI were made under the initiatives of the "ADHD-200 Consortium". Despite significant advances in understanding abnormalities related to brain maturation and function, neuroimaging findings in ADHD research cannot yet be used to support clinical practice due to a variety of concerns.

An alternative tool to assess ADHD worth to explore is functional near-infrared spectroscopy (fNIRS), which is characterized by being noninvasive, wearable, cost-effective, and deployable in more friendly/ecological settings. fNIRS has shown its usefulness in monitoring functional hemodynamic changes associated with cortical brain activation. Compared to other neuroimaging modalities, few fNIRS studies have been conducted to discriminate children with ADHD from healthy controls, some of them trying to improve classification by combining different modalities (e.g. EEG+fNIRS). Even fewer studies focused on single unimodal approaches by using "exclusively" NIRS data. For example, Monden et al (2015, "Individual classification of ADHD children by right prefrontal hemodynamic responses during a go/no-go task as assessed by fNIRS") reported a classification accuracy of 85% with a sensitivity of 90% by analyzing ROC curves obtained from right prefrontal oxy-Hb activation data during a go/no-go task.

Using prefrontal cortex activation measures during an N-back task, Crippa et al. (2017, "The utility of a computerized algorithm based on a multi-domain profile of measures for the diagnosis of attention deficit/hyperactivity disorder") achieved mean accuracies of 78% with 72% sensitivity and 82% specificity when a support vector machine (SVM) classifier was trained on data from deoxy-Hb. Also employing an N-back task and an SVM, Gu et al. (2018, "Identifying ADHD children using hemodynamic responses during a working memory task measured by functional near-infrared spectroscopy") reached 86% of accuracy with oxy-Hb data measured in the prefrontal and temporal cortex]. It is worth noting that no correction for components of non-cerebral origin was applied to the fNIRS signals in the aforementioned studies, which is especially important when scanning the prefrontal cortex through the forehead, since functional extra- and cerebral responses are interrelated processes that overlap in fNIRS recordings and with a greater confounding effect for oxy-Hb.

Notwithstanding this known drawback of fNIRS, classification algorithms can achieve appreciable performance by learning some type of feature representation from the uncorrected NIRS data, but uncertainty about the nature and origin of the features hampers the interpretability of predictive models. In these studies, the features were based on some kind of measurement from the averaged fNIRS data across trials/epochs, a classic approach that, while often providing robust results, fails to uncover finer distinctive patterns embedded in the data.

It is also known that a rhythmic mental arithmetic task successfully induced cyclical hemodynamic fluctuations coupled to the task frequency (33 mHz), and that the oscillatory patterns were consistent across individuals both in superficial and deep fNIRS signals recorded in the frontopolar region. Spectral analysis also showed oscillatory activity at lower frequencies (< 33 mHz) seen at rest and during mental task, and with a prominent peak around 5-10 mHz. Resting-state fMRI studies have reported that ADHD patients show significant differences in the lowfrequency oscillations (LFO; 10-80 mHz) band across multiple brain regions, with separable contribution of specific frequency sub-bands including extra-low frequencies (0-10 mHz). These differences have been related to abnormalities in the salience, attentional and default-mode networks functioning, but the inconsistences observed across many studies point to a large heterogeneity in spontaneous brain activity in ADHD. Despite this, evidence suggests that some characteristics of ADHD brain activity are sensitive to specific frequency bands.

GHORBANIAN P. ET AL: "Continuous Wavelet Transform EEG Features of Alzheimer's Disease", ASME 2012 5TH ANNUAL DYNAMIC SYSTEMS AND CONTROL CONFERENCE JOINT WITH THE JSME 2012 11TH MOTION AND VIBRATION CONFERENCE, 13 January 2015 (2015-01-13), pages 567-572, DOI: 10.1115/ DSCC2012-MOVIC2012-8684ISBN: 978-0-7918-4529-5 teaches the application of the continuous wavelet transform (CWT) to determine EEG discriminating features of Alzheimer's Disease patients compared to control subjects.

Computer-aided diagnosis of attention-deficit/hyperactivity disorder (ADHD) aims to provide useful adjunctive indicators to support more accurate and cost-effective clinical decisions. Deep- and machine-learning (ML) techniques are increasingly used to identify neuroimaging-based features for objective assessment of ADHD. Despite promising results in diagnostic prediction, substantial barriers still hamper the translation of the research into daily clinic. Few studies have focused on functional near-infrared spectroscopy (fNIRS) data to discriminate ADHD condition at the individual level.

It is therefore an objective of the present invention providing a computerimplemented method for selecting functional biomarkers to identify a target condition in a subject, wherein said target condition can be, for example, attentiondeficit/hyperactivity disorder (ADHD).

It is also an objective of the present invention providing a classifier being trained using the selected functional biomarkers, so it can be used for classifying contributing to the objective assessment of target conditions such as ADHD through computer-aided affordable tools deployable in many clinical settings.

## Description of the invention

The computer implemented method of the present invention for selecting functional biomarkers to identify a target condition, such as attentiondeficit/hyperactivity disorder, in a subject comprises the steps of: obtaining groups of equivalent physiological signals generated during a stimulation, comprising a first group of physiological signals from subjects presenting the target condition, and a second group of physiological signals from subjects of control condition, not presenting the target condition; identifying frequency sub-bands for each group of physiological signals corresponding to frequencies that show higher group synchronization; obtaining per each identified frequency sub-band a corresponding time-series pattern; and selecting one or more time-series patterns having similarity values to the physiological signals of each group that differentiate the groups of physiological signals as functional biomarkers to identify the target condition, for example for training a classifier of the target condition for identifying the target condition from a physiological signal obtained from a subject during the same stimulation.

Advantageously, the computer implemented method allows selecting time-series patterns as functional biomarkers to develop a methodological approach for effective identification of a target condition in a subject, such as training a classifier, for example a machine learning classifier of those known in the state of the art. This classifier can then be used in a computer implemented method for identifying if a subject from which an equivalent physiological signal is obtained during the same stimulation is more likely to be a subject presenting the target condition or a subject not presenting the target condition, it is, a subject presenting the control condition.

According to an embodiment of the invention, the stimulation is a periodical task having a task frequency, the frequency sub-bands including the task frequency.

According to an embodiment of the invention, the physiological signal is one of a cerebral signal, such as a NIRS signal, a fMRI-BOLD signal, or an EEG signal, or a hemodynamic signal such as a blood pressure or flow signal. The method can also be performed simultaneously for different physiological signals, therefore obtaining functional biomarkers corresponding to physiological signals of different kind. Moreover, the selected functional biomarkers can be combined for training the classifier.

According to an embodiment of the invention, the physiological signal is a cerebral signal, obtained in a same region of the brain, in response to relative or absolute changes in the concentration of a hemoglobin chromophore, so the cerebral signal can be externally obtained with devices known by the skilled person.

According to an embodiment of the invention, the step of identifying frequency sub-band comprises, per each group: obtaining a time-frequency transform of each physiological signal; averaging the time-frequency transforms; extracting the local-maxima of the averaged time-frequency transforms; and identifying the frequency sub-bands for the group as frequency ranges around the local-maxima using a suitable predefined criteria such as between the local-minima around each local-maxima. Other suitable predefined criteria could also be used, such as setting a percentage of height descent on both sides around the local-maxima, for example, 50% of the local-maxima height. The physiological signals are expected to be adapted as necessary, and also signals obtained from the physiological signals, such as transforms, can also be used. The number of frequency sub-bands can also be different per each group. For example, frequency sub-bands could be limited to those presenting a bandwidth within a certain threshold, or frequency sub-bands with a local-maximum within a certain threshold, or selecting a predefined number of frequency sub-bands based on the best bandwidth and local-maxima values. It is also envisaged, when using multiple physiological signals, to combine frequency sub-bands obtained from multiple physiological signals in case several physiological signals of similar frequency response are used, such as similar cerebral signals obtained from different regions of interest, so the common most relevant frequency sub-bands are identified. For example, averaging frequency sub-bands or selecting the combined maxima of the frequency sub-bands.

According to an embodiment of the invention, the time-frequency transform is a continuous wavelet transform. Other transforms known by the skilled person, such as short-timed Fourier transform, or Wigner-Wille transform could also be used.

According to an embodiment of the invention, the step of identifying frequency sub-bands comprises calculating an inter-subject synchronization measure, and identifying the frequency sub-bands for the group as frequency ranges around the local-maxima. The inter-subject synchronization measure is one of a phase-synchronization measures, or coherence measures, or correlation measures, of the measures known by the skilled person.

According to an embodiment of the invention, the inter-subject synchronization measure comprises a phase synchronization measure.

According to an embodiment of the invention, the inter-subject synchronization measure comprises a combination of a phase and magnitude synchronization measure.

According to an embodiment of the invention, the step of obtaining per each identified frequency sub-band a corresponding time-series pattern per each group comprises: computing the inverse time-frequency transform from the calculated time-frequency transform of the physiological signals per each frequency sub-band to obtain the corresponding, band-limited, time-series for each member of the group; and generating the time-series pattern of the group by averaging the obtained time-series for each member of the group.

According to an embodiment of the invention, the step of selecting the one or more time-series patterns that better differentiate the groups of physiological signals comprises calculating time-series similarity measures, such as Euclidean, Mahalanobis, Cityblock distances or elastic measures, as Dynamic Time Warping, or correlation methods. Similarity is calculated between each candidate time-series pattern and the time-series of each member of the group, and selecting the time-series patterns with similarity measures that better separate the groups, as functional biomarkers.

According to an embodiment of the invention, the best time-series patterns are selected based on statistical contrast at a predefined significance level, such as the F-statistic for the analysis of variance, so only the most relevant time-series patterns are selected, thus improving the computational efficiency of the classifier. Other selection criteria of the best time-series patterns are also envisaged, even no selection is contemplated, using all available time-series patterns.

A classifier for identifying the target condition in a subject trained using the similarity values to the functional biomarkers is also disclosed. The classifier using the selected time-series patterns as functional biomarkers allows classifying if a physiological signal of a subject is likely to be a physiological signal from a subject presenting the target condition or from subjects of control condition, not presenting the target condition.

A computer implemented method for identifying the target condition in a subject using the trained classifier is also disclosed, comprising obtaining the equivalent physiological signal from a subject during the same stimulation previously used for selecting the functional biomarkers; and processing the physiological signal in the classifier for identifying the target condition in the subject. Also, computer programs including instructions that when executed cause a machine to perform the previous computer implemented methods, are disclosed.

Brief description of the drawings

As a complement to the description provided herein and for the purpose of helping to make the characteristics of the invention more readily understandable, this specification is accompanied by a set of drawings, which by the way of illustration and not limitation, represent the following:

Fig. 1 presents a general schema of the computer implemented method of the present invention for selecting functional biomarkers F;

Fig. 2 presents a general schema of the computer implemented method of the present invention for training a classifier using the selected functional biomarkers F.

Fig. 3 presents a general schema of the computer implemented method of the present invention detecting a target condition in a subject using the trained classifier.

Fig. 4 presents a schematic of a stimulation or task for obtaining physiological signals from a subject;

Fig. 5 presents a setup for obtaining physiological signals from a subject, in this case, the physiological signals corresponding to cerebral signals from three regions of interest, right., medial, left using a known device;

Fig. 6 presents a physiological signal obtained from a subject and the corresponding continuous wavelet transform (CWT) of the physiological signal.

Fig. 7a presents the schematics of the procedure to compute a representative inter-subject synchronization ISS map, from physiological signals of a same group of subjects, presenting o not presenting the target condition;

Fig. 7b presents the inter-subject synchronization ISS of Fig. 7a and its frequency sub-bands;

Fig. 8 presents an schematic example of the procedure to extract a candidate time-series pattern from the physiological signals of a group of subjects, and comparing the candidate time-series pattern with time-series obtained from the physiological signals of all groups of subjects for determining if the candidate time-series pattern differentiates the groups of physiological signals;

Fig. 9a presents the inter-subject synchronization of the physiological signals for shallow-signals for the three regions of interest, right, medial, left obtained with the setup of Fig. 5;

Fig. 9b presents inter-subject synchronization of the physiological signals for clean-signals CS for the three regions of interest, right, medial, left obtained with the setup of Fig. 5;

Fig. 10 presents the average inter-subject synchronization patterns across frequencies of the three regions of interest, for shallow-signals and clean-signals, for HbO and HbR chromophores of the group of subjects presenting the target condition and the group of subjects not presenting the target condition, and the corresponding frequency sub-bands.

Fig. 11a presents time-series pattern candidates for shallow signals (SS) for each frequency sub-band, each region of interest (right, medial, left), and each chromophore (HbO -upper-, HbR -lower-) from the group of subjects presenting the target condition and the group of subjects not presenting the target condition;

Fig. 11b presents time-series pattern candidates for clean signals (CS) for each frequency sub-band, each region of interest (right, medial, left), and each chromophore (HbO -upper-, HbR -lower-) from the group of subjects presenting the target condition and the group of subjects not presenting the target condition; and

Fig. 12 presents performance scores achieved by each classification model trained using the selected time-series patterns.

Detailed description of an embodiment of the invention

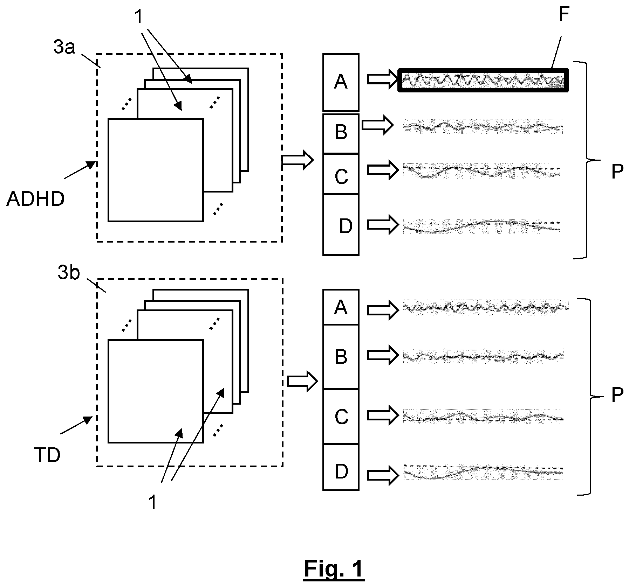

Fig. 1 presents a general schema of the computer implemented method of the present invention for selecting functional biomarkers F to identify a target condition ADHD in a subject 2. As it can be seen, the method comprising the steps of obtaining groups of equivalent physiological signals 1, generated during a stimulation S, comprising a first group 3a of physiological signals 1 from subjects 2 presenting the target condition ADHD, and a second group 3b of physiological signals 1 from subjects 2 of control condition TD, not presenting the target condition ADHD. As it will be later described, the stimulation S can be a periodical task having a task frequency, the frequency sub-band A,B,C,D including the task frequency. The physiological signal 1 can be a cerebral signal, such as a NIRS signal, a fMRI-BOLD signal, or an EEG signal, or an hemodynamic signal such as a blood pressure or flow signal. As it will be later shown, the physiological signal 1 can be a cerebral signal, obtained in a same region of the brain, in response to relative or absolute changes in the concentration of a hemoglobin chromophore. It is expected that the method described could be programmed and executed by a computer, for example a local, remote, distributed computer or an embedded device. Further, a program including instructions that when executed cause a machine to perform the methods described are envisaged.

As shown in Fig. 1, the method also comprises, for each group 3a,3b of physiological signals 1, a step of identifying frequency sub-bands A,B,C,D corresponding to frequencies that show higher group synchronization. Therefore, each group 3a,3b will be associated with certain frequency sub-bands A,B,C,D. As also shown, the method further comprises a step of obtaining per each identified frequency sub-band A,B,C,D, of each group 3a,3b a corresponding time-series pattern P.

Once a time-series pattern P is obtained per each frequency sub-band A,B,C,D of each group 3a,3b, the method further comprises selecting the one or more representative time-series patterns P having similarity values d to the physiological signals 1 of each group that differentiate the groups of physiological signals 1 as functional biomarkers F to identify a target condition ADHD, that can be used for training a classifier CL of the target condition ADHD.

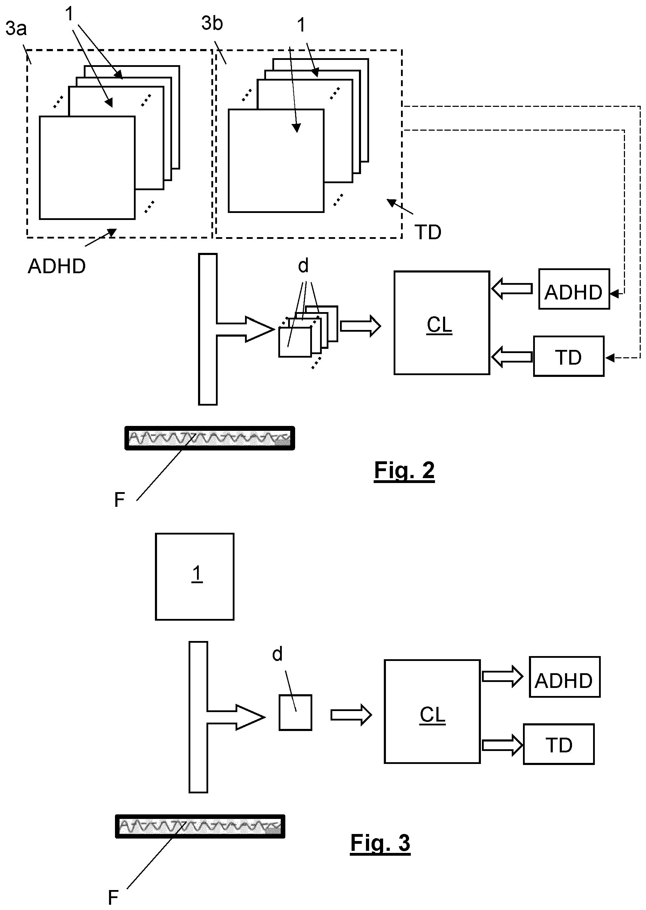

Fig. 2 presents a schema of the training of a classifier CL for the target condition ADHD and control condition TD using the similarity values d to the selected functional biomarkers F of the previous physiological signals 1 of each group 3a,3b. The classifier CL is trained using supervised learning, each similarity value d being fed to the classifier CL indicating if corresponds to a physiological signal 1 of the first group 3a, presenting the target condition ADHD, or to a physiological signal 1 of the second group 3b, presenting a control condition TD and not presenting the target condition ADHD. This information can be fed as a probability of the physiological signal 1 being of the first group 3a, thus having the target condition ADHD.

Fig. 3 presents a schema of a computer implemented method for identifying a target condition ADHD in a subject 2 comprising obtaining a physiological signal 1 from the subject 2 during a stimulation S; and processing the physiological signal 1 in the classifier CL for identifying the target condition ADHD in the subject 2. The physiological signal 1 and the stimulation S must be equivalent to the physiological signal 1 and stimulation S used during training, so the obtained physiological signal 1 from the subject 2 can be classified. For example, the output of the classifier CL can be a probability of the physiological signal 1 being of the first group 3a, thus having the target condition ADHD. If this probability is higher than a threshold, also a binary output can be given, thus informing that the subject 2 is likely to show the target condition ADHD.

A detailed embodiment of the invention related to a computer implemented method for selecting functional biomarkers to identify a target condition of ADHD in a subject will be explained thereafter. Naturally, other target mental or brain disorder conditions, target body physiological conditions or combination of them could be used as a target condition for selecting the corresponding functional biomarkers for that target condition.

In this embodiment, groups of equivalent physiological signals generated during a stimulation S will be obtained, comprising a first group 3a of physiological signals 1 from subjects presenting the target condition ADHD, and a second group 3b of physiological signals from subjects of control condition TD, not presenting the target condition. Although a single equivalent physiological signal could be used, in the described embodiment several equivalent physiological signals will be used, so functional biomarkers could be extracted from each of the equivalent physiological signals. In the embodiment, two kind cerebral signals (shallow-signal SS and clean-signal CS), from three different regions of interest (left, medium, central) and corresponding to the concentration of two chromophores, therefore, twelve equivalent physiological signals will be used for obtaining the functional biomarkers F for each equivalent signal that separate the group of subjects presenting the target condition (ADHD) and the group of subjects of control condition (TD), not presenting the target condition.

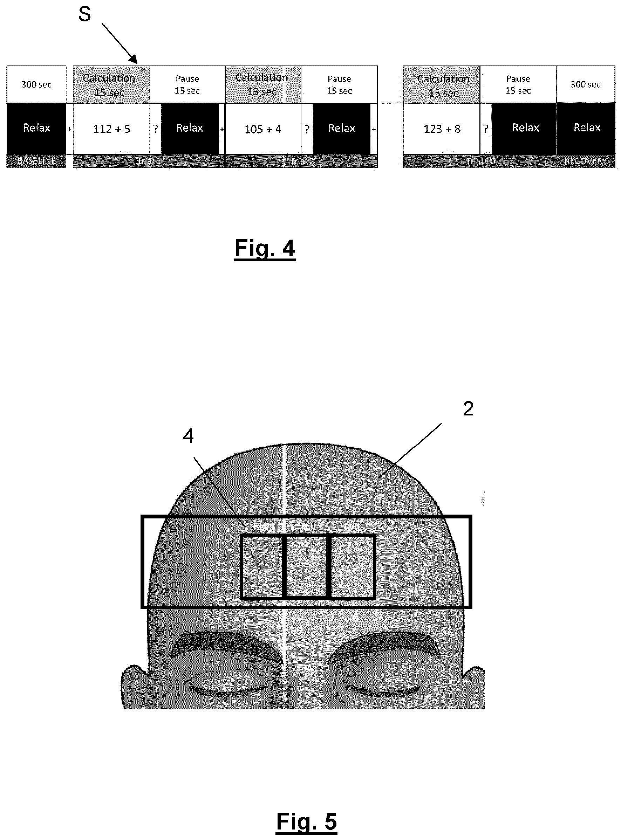

Fig. 4 presents an example of a stimulation that can be used for the computer implemented method for selecting functional biomarkers to identify a target condition of the present invention. In this case this stimulation is a mental or cognitive activity that can be programmed in a computer comprising 10 consecutive 30 seconds trials, each starting with 15 seconds of mental calculation followed by a 15 seconds pause of relaxation. During mental math, participants were asked to iteratively add a small number (5 to 9) to a three-digit number (100 to 199) (both numbers randomly chosen), silently and as quickly and accurately as possible. The pause then begins by presenting the question "Result?" for 5 seconds, prompting for the voicing of the final result reached, followed by a black screen indicating mental relaxation until a 2 seconds fixation cross announced the start of the next trial. The task lasted 300 seconds and was uninterruptedly preceded by 300 seconds of baseline recording in resting state and followed by another 300 seconds of recovery in relaxed state. The 30 seconds period of the trials corresponds to a frequency of 0.033 Hz, which will be referred to as the task frequency. We also note that this design minimizes speech during the task, thus avoiding significant changes in breathing that could affect cerebral hemodynamics. Naturally, other stimulations could be used instead, as long as they can be reproduced for obtaining the physiological signals when selecting the functional biomarkers and when classifying using the trained classifier. Even non periodical tasks of stimulations could be used as long as they can be reproduced. Not only mental tasks are considered, also physical tasks are contemplated, for example body tilting or physical exercises, that could also be periodic, as periodically raising an arm.

For recording and processing the physiological signal, as a fNIRS signal, analysis were performed on superficial and regression-corrected deep fNIRS signals recorded from the forehead through a multi-distance, multi-channel device such as the device 4 disclosed in Fig. 5 from Tehia, Newmanbrain, S.L., Elche, Spain also described in Molina-Rodriguez S et al. (2022, "Frequency-domain analysis of fNIRS fluctuations induced by rhythmic mental arithmetic Psychophysiology") .

Using such device 4 , relative concentration changes in oxy- (HbO) and deoxy-hemoglobin (HbR) were computed in a known matter for different regions of interest, namely a left, middle and right regions, therefore physiological signals can be external cerebral signals from three regions of interest (ROI), right., medial, left, to different chromophores HbO HbR and different signal type (shallow-signal -SS- and clean-signal -CS-), it is, twelve physiological signals can be considered

Identification of the frequency components with potential capacity to discriminate between the two groups of participants (i.e., having the control condition TD and having the target condition ADHD) was a crucial issue, as it will be discussed afterwards.

To this end, a suitable method for locating stimulation or task-related oscillations was needed on different time scales (i.e., frequency bands), appropriate for non-stationary signal analysis, and capable of providing some measure of similarity to define class membership. A data-driven approach based on time-frequency transform, as complex continuous wavelet transform CWT, and time-scale synchronization detection was used.

CWT is an time-frequency transform signal processing method that provides a time-frequency (or time-scale) representation of the characteristics of a signal on the basis of the dilation and translation of a mother wavelet function; theory and mathematical description can be found elsewhere. CWT can be viewed as a bandpass filter with varying bandwidths automatically defined by the wavelet scale, which avoids the drawbacks of using custom filters.

To compute the complex continuous wavelet transform CWT generalized Morse wavelets were used, a flexible superfamily of exactly analytic wavelets particularly useful for analyzing signals with time-varying amplitude and frequency, i.e. modulated signals. Since Morse wavelets can be tuned to encompass many other analytic wavelets commonly used, they provide a unified framework as reference point.

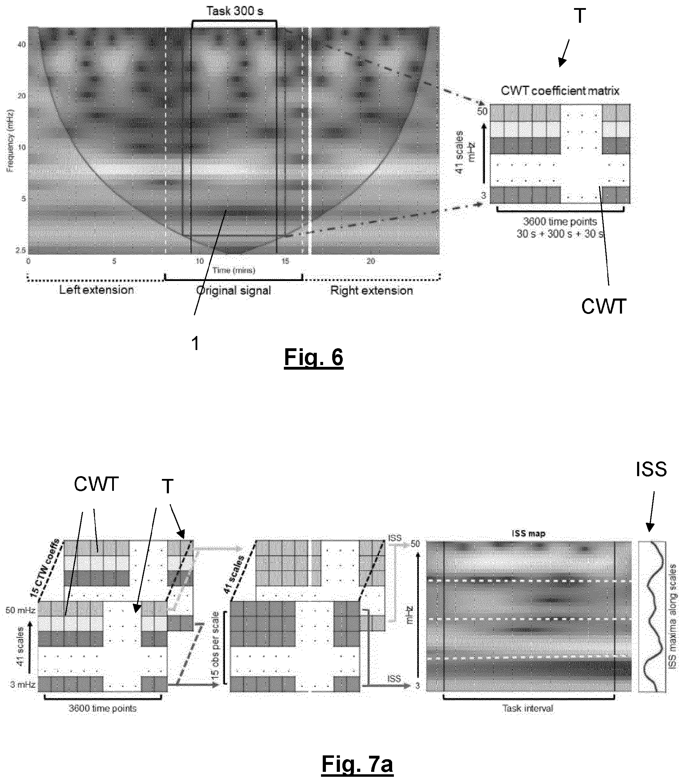

Depending on the kind of physiological signal, the range of frequencies of interest can be adapted, for example for NIRS or fMIR being up to 0 to 100mHz; EEG being up to 0 to 40Hz; and electromyogram being up to 0 to 600Hz. Also the range of frequencies of interest will be determined by the Nyquist frequency of the sampling rate, as known by the skilled person. In the presented embodiment, also a preliminary PSD analysis showed that most of the spectral power was within the 0 - 50 mHz band, the CWT was focused on that frequency range. As known CWT was applied to the symmetrically extended signals with the scale discretization parameter voices-per-octave = 10, which after calculation of the minimum and maximum allowable bandpass results in 45 scales with approximate frequencies ranging from 2.4 to 50 mHz.. Thus, in this case the number of usable scales was limited to 41 (3 to 50 mHz), as disclosed in Fig. 6.

Fig. 6 shows a first part of the step of identifying frequency sub-band A,B,C,D per each group 3a,3b. In Fig. 4 it can be seen that a time-frequency transform T is obtained of each physiological signal 1, as a continuous wavelet transform (CWT). Fig. 6 shown a schematic of a continuous wavelet transform CWT of deep HbO signal from mid-ROI. Also Fig. 6 presents a scalogram of the symmetrically extended data with the cone-of-influence. White dashed lines enclose the original physiological signal and the solid black lines delimit the taskperiod. A box depicts the scalogram region from which the coefficients for analysis were extracted. Further schematics of the extracted coefficient matrix containing 41 scales and 3600 time points corresponding to the box is presented. After CWT, the full coefficient matrix is kept for later computation of the inverse CWT (i.e. including the data corresponding to the extended segments) whereas for the next step we would use only a shorter portion of the matrix. Under the hypothesis that the stimulation, as a math task, may induce differentiated fNIRS fluctuations for the control condition TD and target condition ADHD groups, the frequency sub-bands that showed higher group synchronizations during the task must be identified.

Since group-wise synchronization may appear as transient peaks rather than constantly, a time-point-by-time-point analysis was performed, which allows capturing common oscillatory patterns that evolve dynamically over time. It is worth noting that this strategy is inspired by the underlying logic of so-called inter-subject correlation (ISC) analysis, a data-driven approach devoted to assessing consistent neural responses to stimuli across.

Instantaneous inter-subject synchronization (ISS) was measured using the magnitude and phase information provided by the complex-valued CWT coefficients. In fMRI studies, measures as inter-subject phase synchronization and pairwise phase consistency have been validated for the assessment of voxel-wise instantaneous phase synchronization across subjects. However, these measures rely only on the uniformity of phase angles, ignoring the magnitude. Thus, when applied to fNIRS it means that low-amplitude signals affect the measurement the same as those with significant amplitude. Therefore, this approach may not be entirely appropriated for fNIRS data where amplitude changes are related to the magnitude of the hemodynamic response. Since as amplitude increases, the signal-to-noise ratio improves, it is reasonable to argue that observations with higher amplitudes can contribute to a more realistic estimate of phase synchronization. Under this assumption, a closely related measure called 'intertrial linear coherence' was used, that combines magnitude and phase in the normalization step. Since the measurement here was across subject observations and not across trials, this measurement is called throughout the invention 'inter-subject synchronization' (ISS) measure, omitting 'linear' for simplicity and similarly, that comprises a combination of a phase and magnitude synchronization measure, formulated as:

$$ISS(f,t) = \frac{\sum_{k=1}^{n} F_k(f,t)}{\sqrt{n \sum_{k=1}^{n} |F_k(f,t)|^2}},$$

where $F_k(f,t)$ is the spectral estimate of observation $k$ at frequency $f$ and time $t$, and the modulus $\|$ represents the complex norm. ISS also takes values between 0 (absence of sync) and 1 (perfect sync).

ISS was computed moment-to-moment for each scale from the CWTs coefficient matrices of each group (i.e. 15 participant observations per scale). The analysis was limited to only to the time-interval between -30 seconds and +30 seconds around the task. This procedure was applied independently to the shallow-signal SS and clean-signal CS data of each group of subjects for each chromophore and ROI, obtaining an ISS representation in the time-frequency plane that can also be visualized as a color map in Fig. 7a.

The maximum ISS observed along each scale was chosen, which represents the highest group synchronization achieved at each specific frequency, as can be also seen in Fig. 7a.

Fig. 7a, shows a part of the step of identifying frequency sub-band A,B,C,D per each group 3a,3b, once a time-frequency transform T as a continuous wavelet transform CWT is obtained for each physiological signal 1. This part comprises averaging the time-frequency transforms T of the group 3a,3b, calculating an inter-subject synchronization ISS measure that can be a phase synchronization measure, and also a combination of a phase and magnitude synchronization measure.

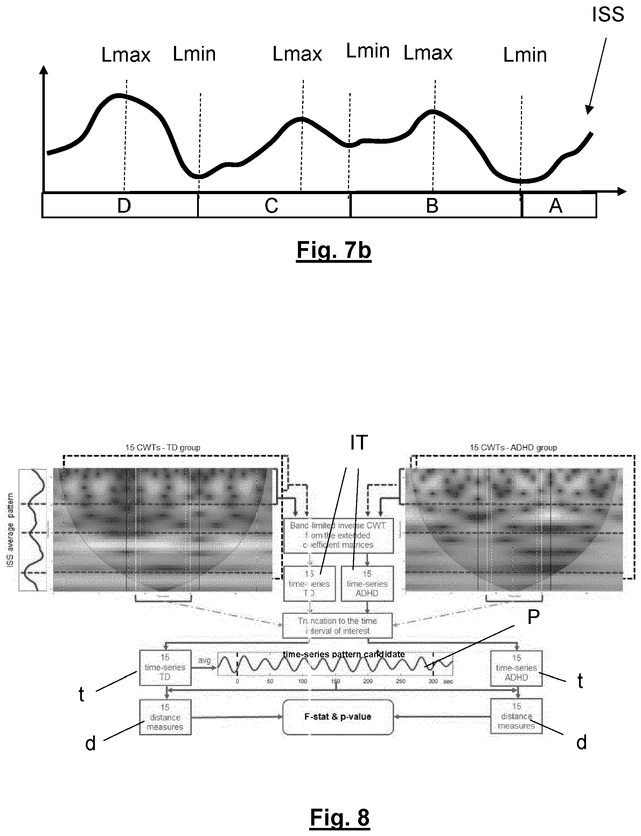

Therefore, in Fig. 7a. schematics of the procedure to compute a representative inter-subject synchronization (ISS) map can be observed. Starting with the coefficient matrices of each individual within a group (left), complex-valued data are put together for each frequency bin (middle) and the ISS is computed moment-to-moment to obtain a representation of the within-group synchronization strength at each frequency over time (right colormap). The rightmost plot depicts the maxima and minima reached by the ISS along frequencies, which are used to delimit the sub-bands to analyze (horizontal white dashed lines), shown in Fig. 7b.

As shown in Fig. 7b, the local-maxima Lmax is extracted from the averaged time-frequency transforms T of the group 3a,3b, calculated as the inter-subject synchronization ISS, and the frequency sub-bands A,B,C,D for the group are identified as frequency ranges around the local-maxima Lmax, in this case between local-minima Lmin around a local-maximum Lmax.

Once frequency sub-bands A,B,C,D are identified, the computer implemented method further comprises obtaining per each identified frequency sub-band A,B,C,D a corresponding time-series pattern P and selecting the one or more representative time-series patterns P having similarity values d to the physiological signals 1 of each group that differentiate the groups of physiological signals 1 as functional biomarkers F to identify the target condition ADHD, and that can be used during the training of a classifier CL of the target condition ADHD. This step comprises computing an inverse time-frequency transform IT from the calculated time-frequency transform T of the physiological signals 1 per each frequency sub-band A,B,C,D to obtain a corresponding time-series t for each member of the group, it is, for each subject 2 of the group 3a,3b; and generating a time-series pattern P of the group by averaging the obtained inverse time-frequency transform IT for each member of the group.

Once one or more time-series patterns P are obtained, selecting the one or more time-series patterns P that better differentiate the groups of physiological signals 1 comprises calculating time-series similarity values d between each candidate time-series pattern P and the time-series t of each member of the group, and selecting the time-series patterns P with similarity values d that better differentiate the groups as functional biomarkers F. The time-series patterns P are selected as functional biomarkers F based on statistical contrast at a predefined significance level.

Like the other aforementioned synchronization measures, ISS is a compound measure that does not exist on its own at a single-subject level but represents a summary statistic of group synchronization. Therefore, to disentangle the contribution to ISS of each individual is not a straightforward issue.

However, ISS peaks suggest that some frequency components show similar time courses across individuals, at least within certain time intervals. In other words, there are sequential patterns common to the group that can provide distinctive information to define class membership. This concept falls into the interdisciplinary and much-studied field of time-series classification, which encompasses a variety of techniques for identifying those properties (features) that have sufficient discriminating power to distinguish between different classes of time series. In the context of the present invention, a well-suited technique could be the one based on the shapelet framework, which addresses the classification problem by discovering primitive time-series sequences (shapelets) that are used to quantify the similarity between classes of time series. Shapelets provide directly interpretable information about patterns (shapes) that are important for understanding how data classes differ, a desirable property for clinical decision support systems.

The basics of the shapelets technique was applied, but instead of looking for phase-independent subsequences similar in shape (i.e. subsequences may be located anywhere in the series), the analysis within a fixed time-interval was performed, all subsequences having the same length. Subsequence translation over time was not applied, which implies that time-series similarity also depends on the phase (i.e. on a consistent time-alignment). Therefore, instead of local, global patterns present over a whole time interval were captured. Under this approach, the term "shapelet" may not be appropriate and the term time-series pattern is used instead.

At this point, we need to extract the time-series to be used for identifying representative time-series patterns P. The average ISS patterns suggest the frequencies that are likely to contain synchronized oscillations. By computing the inverse time-frequency transform IT as inverse CWT within the specific frequency sub-band A,B,C,D defined by the bounds of an ISS peak, a band-limited components in the time-domain can be reconstructed. To reduce edge-effects, the inverse CWT was computed from the extended coefficient matrix that we reserved in a previous step. Then, the resulting time-series were truncated to the interval between -30 seconds and +30 seconds around the task. After applying this procedure to the CWT of all the individuals belonging to a group, a set of time-series (n = 15) is available to find a reference time-series pattern for that group in a particular sub-band.

Since all the time-series have the same length and are within the same timeframe, a suitable reference time-series pattern can be obtained simply by averaging. If the time-series share a common pattern, their average should represent the group well enough. To quantify similarity with the reference time-series pattern, among other possibilities, a simple measure as Euclidean distance can be computed:

$$D(S,T) = \sqrt{\sum_{i=1}^{n}(S_i - T_i)^2}$$

where *S* **denotes** the reference time-series pattern and *T* a time-series, both of length *n*. Note that *S* and *T* should be standardized to have mean 0 and standard deviation 1, which ensures to operate on the same scale. Standardization also allows us to relate the Euclidean distance to the correlation coefficient ($r=1-D^2/2n$).

In order to assess the capability of the time-series pattern to discriminate between groups, the distances obtained from one group with those of the other group were contrasted. For example, a time-series pattern that is representative of the control condition TD group should have smaller distances to members of this group than to members of the target condition ADHD group, and vice versa.

Among other quality measures, the F-statistic for analysis of variance can be used to assess the discriminative power of a time-series pattern. This statistic indicates the ratio of the between-group variability to the within-group variability as:

$$F = \frac{\sum_{i=1}^{C}\dfrac{(\overline{D}_i - \overline{D})^2}{C-1}}{\sum_{i=1}^{C}\sum_{d_j \in D_i}\dfrac{(d_j - \overline{D}_i)^2}{n-C}}$$

where C is the number of classes (or groups; in our case = 2), $\overline{D}$ is the overall mean of all distances, $\overline{D}_i$ is the mean of the distances within class *i,* and *n* is the number of time-series. The better the time-series pattern the greater the F value, because the difference between-groups increases while it decreases within a group. The corresponding p-value can be calculated from the F-distribution.

Based on the average ISS patterns, four candidate frequency sub-bands A,B,C,D were identified in each of them that were labeled A, B, C and D in decreasing order of frequency. Each sub-band contains a peak (local-maximum) flanked by two troughs (local-minima) that delimit the frequency boundaries. For each ISS pattern, each belonging to a target group (TD or ADHD), the following procedure for each sub-band was performed, as indicated in Fig. 8: (i) Compute the inverse CWT within the sub-band from the extended coefficient matrices of both groups to obtain the corresponding time-series (n = 15 + 15 = 30). (ii) Truncate time-series to the time-interval of interest. (iii) Generate the reference time-series pattern by averaging only the time-series of the target group (n = 15), a variability measure such as the standard error of the mean (SEM) can also be computed. (iv) Calculate distances to the time-series pattern for the time-series of both groups. (v) Calculate F and p-value from the 15 + 15 = 30 distances. Because each ISS pattern and its sub-bands are common to all three ROIs, this procedure was applied separately to each ROI data but using the same sub-bands. Thus, for each ISS pattern we obtain a matrix of 30 x 12 distances, where each row contains the distance measures of an individual and columns correspond to the 4 sub-bands x 3 ROIs. Since there are eight ISS patterns, the final matrix was of size 30 x 96, 48 columns for SS and 48 for CS, while 15 rows correspond to the TD group and 15 to ADHD. Please note that each column has an associated F-stat and p-value, indicating how well a particular time-series pattern differentiates the groups.

In summary, the time-series pattern approach as employed to transform data observations at different time-scales into a simple feature space of Euclidean distances, but other feature type known in the state of the art could be used

Fig. 8 shows therefore a schematic example of the procedure to extract reference time-series patterns for the control condition TD group and contrast them with the target condition ADHD group. Per-subject time-series within a specific frequency sub-band A,B,C,D are obtained from their band-limited inverse-CWT and averaged only for the control condition TD group to get the time-series pattern. Euclidean distances to the time-series pattern are computed for the time-series of both groups and then within- and between-group variances are contrasted to obtain the F-stat value.

To assess the feasibility of the proposed procedure to differentiate between control condition TD and target condition ADHD, four well-suited classifiers CL, machine learning algorithms for supervised binary classification, namely linear support vector machine (SVM), logistic regression (LR), linear discriminant analysis (LDA) and Gaussian naïve Bayes (NB) were tested.

These algorithms were selected because they are well known, inherently interpretable, computationally efficient, and can work with relatively small sample sizes. Under a variety of flavors (different kernel, regularization, etc.), SVM is very frequently present in neuroimaging-based studies of brain disorders, with LDA and LR being the other most popular choices. Although less commonly used, we included NB because its ease of application and good performance in a variety of applications despite the assumption of feature independence.

SVM differentiate classes by finding the hyperplane that maximizes the separation (margin) between the points of them. LR do the job through a logistic function (sigmoid) that model the dependent variable and maps predicted values into probabilities of belonging to one class or another. LDA assumes that the predictors come from a Gaussian mixture distribution and uses discriminant functions to estimate the probability that they are from each class. NB estimates the probability density of predictors given a class by independently mapping them onto separate normal distributions fitted to each class. Although based on different models, discriminative (LR & SVM) vs generative (LDA & NB), all four are within the linear classifier category, i.e. to make predictions, the classifiers try to learn the line that best separates the points of the two classes.

In addition to the putative functional response, fNIRS signals also contain components originating from common systemic forces and unpredictable local activity. Therefore, it is very likely that the feature matrix also contains redundant and/or irrelevant data that can degrade classifier performance by cause of overfitting and noise issues. Model regularization can be applied to some algorithms to account for statistical overfitting, however that raises the problem of choosing a suitable technique (e.g. lasso) and finding appropriate regularization parameters. To avoid increasing the complexity of the models, the problem was addressed by reducing the feature space. Feature selection is a commonly used tool to obtain a smaller subset of the most relevant features, reducing complexity while improving classification accuracy and generalization capacity. A reasonable hybrid approach is to first apply a filter method, before modeling, to select some features based only on their intrinsic properties. Then more sophisticated methods such as wrappers may be employed to find the best subset of features, using the classifier itself as evaluator. Among others, a benefit of such selection is an easier explanation of the prediction because the models are simpler.

A wide variety of filter methods have been developed, each based on specific criteria (information, similarity, etc.) to evaluate features. Despite the ample offer, and after trying some of the popular ones (relief, minimum redundancy-maximum relevance and chi-square; results not shown), we settled on a fairly straight option based on the F-statistic. We simply selected the features that showed p-values < .01, assuming that their generating time-series patterns were very unlikely to separate the groups by chance. In this way, we significantly reduced the features from 48 to 5 for SS and from 48 to 10 for CS.

The classification methods were first applied to filter-selected features separately for SS and CS, and then for all of them together (SS + CS). To assess the predictive performance we applied two cross-validation (CV) techniques for comparison purposes, namely leave-one-out (LOO) and stratified 5-fold. In the first, data was partitioned into 30 folds where each observation was used once as a test set and the remaining ones formed the training set. In the latter, five partitions were randomly chosen, each with 26 observations as the training set and 6 as the test set; folds were repartitioned over 20 Monte-Carlo repetitions (5 x 20 = 100 models) to reduce CV variance, while stratification ensured that sets had the same proportion of classes (50% in our case). We used 5- instead of 10-fold because with the latter the test set size = 3 would be too close to that of LOO = 1. Since we are dealing with only two classes and our datasets are well-balanced (i.e. equal proportion of both classes), accuracy, specificity and sensitivity can be used as metrics to assess performance, as obtained from the corresponding confusion matrices and then averaged across folds. At this point, we focused on accuracy (a commonly used metric in practice) to test the statistically significant classification performance. Thus, we computed the theoretical above-chance accuracy threshold based on the binomial cumulative distribution at p < 10-3 for 2-classes (probability = 0.5) and a sample size = 30.

Afterwards, a wrapper method can be applied to fine-tune the feature selection such as a Sequential Forward Floating Selection (SFFS) algorithm. SFFS starts with an empty set and sequentially adds one feature at a time to create candidate subsets that are evaluated by cross-validation. After that, the best feature is added to the set. When the size of the selected set is > 2, a backward step tries to optimize it by removing one or more features. This procedure is repeated until there is no performance improvement. The two aforementioned cross-validation techniques were also applied independently to each classifier. Noteworthy, the input order of the finally selected features allows us to know their relative importance. It's also worth mentioning that our wrapper can rank features by multiple metrics at the same time, and that in this work we used two criteria to select/remove features, specificity and then accuracy. Thus, if two (or more) features equally improve the specificity, the one with the best accuracy is selected.

Once the best subset of features was selected for each classifier, we estimated the statistical significance of the observed performance through a nonparametric label permutation procedure that does not assume any particular statistical property of the data. We generated 5000 resamples, each of which randomly permuted the labels of the two classes; realizing the null hypothesis that features do not define class membership. For each resampled data, the classification performance was evaluated using the same cross-validation scheme as for the real data. The observed performance metrics were ranked against the corresponding null-distribution to estimate a p-value. In addition, a 95% bias corrected percentile interval was estimated as confidence interval for each metric by bootstrapping (with replacement) over 2000 resamples, with each realization keeping the same proportion of classes (50%) and at least three distinct observations in each class.

Finally, we checked whether the wrapper performance might have been biased due to the use of a pre- filtered feature set that included all data in the selection process, i.e. the "peeking" effect. To this end, we repeated the wrapper procedure but using the full feature set (i.e. 96 features), then comparing the performance outcomes.

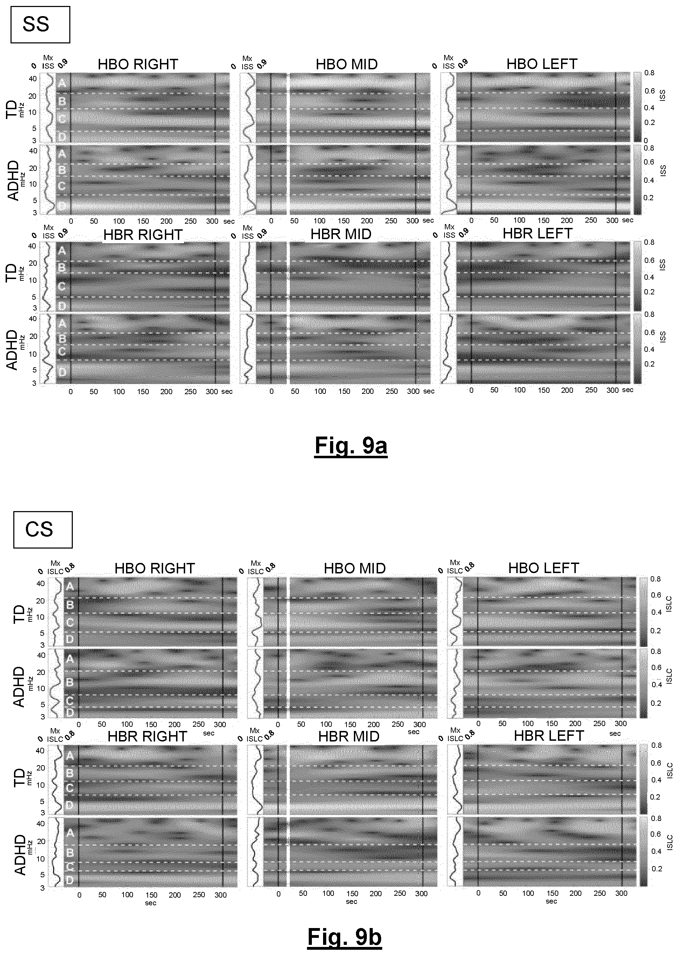

Fig. 9a shows the ISS representation in the time-frequency plane obtained from the complex CWTs of the SS data for both groups; within the range of 3 to 50 mHz. Similarly, Fig. 9b depicts the ISS maps corresponding to the CS data. At first glance, well-defined synchronization zones can be seen within certain frequency sub-bands, some similar in both groups and others clearly differentiated. Here some of them as examples are highlighted. Regarding SS, strong synchronization can be seen in all ROIs around 33 mHz for HbO of TD group, and in right- and mid-ROI at 7 mHz and bellow 4 mHz. Noteworthy, the ADHD group presents even stronger ISS around 4 mHz in all ROIs, but much less evident at 7 or 33 mHz. Regarding CS, albeit to a lesser extent, TD group also synchronizes at 33 mHz while TD group does so in a more diffuse and weak way. Furthermore, TD group seems to be more synchronized during the first part of the task at 7 mHz (mid- and left-ROI), whereas the ADHD group is synchronized in the last part around 17 mHz. Yet another remarkable sync is observed for the HbR of TD group at 4 mHz. Overall, ISS analysis reveals a plurality of sub-bands that can carry information about similarities and differences between groups.

Fig. 9a represents therefore inter-subject synchronization (ISS) color maps for shallow-signals SS within each ROI. Upper rows correspond to HbO for TD and ADHD groups, while lower rows refer to HbR. The small plots to the left of each map depict the ISS maxima across frequencies for each case. Vertical black lines delimit the task-interval. Horizontal white dashed lines delimit the common sub-bands obtained by averaging ISS maxima across ROIs. Labels A-D identify each of these sub-bands. Fig. 9b represents inter-subject synchronization (ISS) color maps for clean-signals CS in a similar manner that in Fig. 9a.

Based on the observed local-maxima Lmax, on each ISS map of Figs. 9a and 9b, several peaks were identified within specific frequency sub-bands A,B,C,D that contain oscillatory components showing some synchronization at the group level. Since such components were expected to provide information to differentiate between groups, it was necessary to locate the frequency sub-bands A,B,C,D exhibiting more discriminative power.

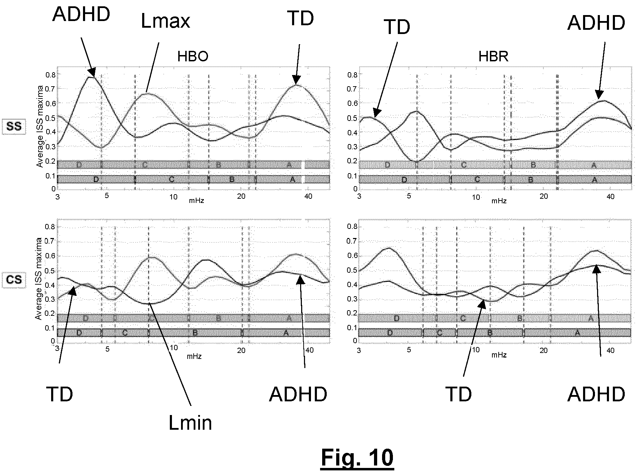

To simplify the procedure, for each case we averaged the ISS data across the three ROIs to obtain the mean ISS maxima per frequency, as shown in Fig. 10. Thus, we simplified the analysis to only one common ISS pattern by chromophore and signal type for each of the two groups, i.e. 2 groups (TD, ADHD) x 2 chromophores (HbO, HbR) x 2 signal types (SS, CS) = 8 ISS patterns. Finally, to reduce noise the ISS patterns were smoothed by moving average using a sliding window of length half the voices-per-octave (i.e. 10/2 = 5). These patterns were examined in the following steps to identify the most relevant frequency components.

Table 1 shows the common synchronization sub-bands estimated from the average ISS maxima across ROIs, labeled A, B, C and D by decreasing frequency, with A corresponding to the task frequency.

Table 1. Frequency bounds of each average sub-band A to D for shallow-signals (SS) and clean-signals (CS), and for each chromophore and group.

| Signal type | Chromophore | Group | Frequency sub -band (mHz) | | | |
|---|---|---|---|---|---|---|
| | | | A | B | C | D |
| SS | HbO | TD | 50 - 21.8 | 21.8 - 11.7 | 11.7-4.7 | 4.7 - 3.0 |
| | | ADHD | 50 - 23.3 | 23.3 - 14.3 | 14.3 - 6.7 | 6.7 - 3.0 |
| | HbR | TD | 50 - 23.0 | 23.0 - 14.3 | 14.3 - 5.4 | 5.4 - 3.0 |
| | | ADHD | 50 - 23.0 | 23.0 - 14.3 | 14.3 - 7.7 | 7.7 - 3.0 |
| CS | HbO | TD | 50 - 21.8 | 21.8 - 11.7 | 11.7 - 5.4 | 5.4 - 3.0 |
| | | ADHD | 50 - 20.3 | 20.3 - 7.7 | 7.7 - 4.7 | 4.7 - 3.0 |
| | HbR | TD | 50 - 21.8 | 21.8 - 11.7 | 11.7 - 6.7 | 6.7 - 3.0 |
| | | ADHD | 50 - 16.5 | 16.5 - 8.2 | 8.2 - 5.8 | 5.8 - 3.0 |

Fig. 10 illustrates how these frequency sub-bands A,B,C,D were delineated by locating the ISS local-minima Lmin surrounding each peak or local-maximum Lmax. Higher peaks can be seen in sub-bands A and C for HbO of TD group in both SS and CS, while ADHD group shows notable peaks in D for SS and B for CS. Regarding HbR, it shows clear peaks in A and D in all cases. Note that within the same assigned sub-band, in some cases the peaks are clearly shifted in frequency depending on the group (e.g. C sub-band for CS-HbO). It is evident again that the ISS maxima also reveal differences at certain frequencies. Fig. 10 therefore discloses the average inter-subject synchronization (ISS) patterns across frequencies. The top row corresponds to shallow-signals (SS) and bottom row to clean-signals (CS). Left and right traces relate respectively to HbO and HbR, with the TD group data and ADHD data. Horizontal labeled rectangles identify each frequency sub-band A,B,C,D, whose boundaries are defined by the local-minima Lmin marked by vertical dashed lines.

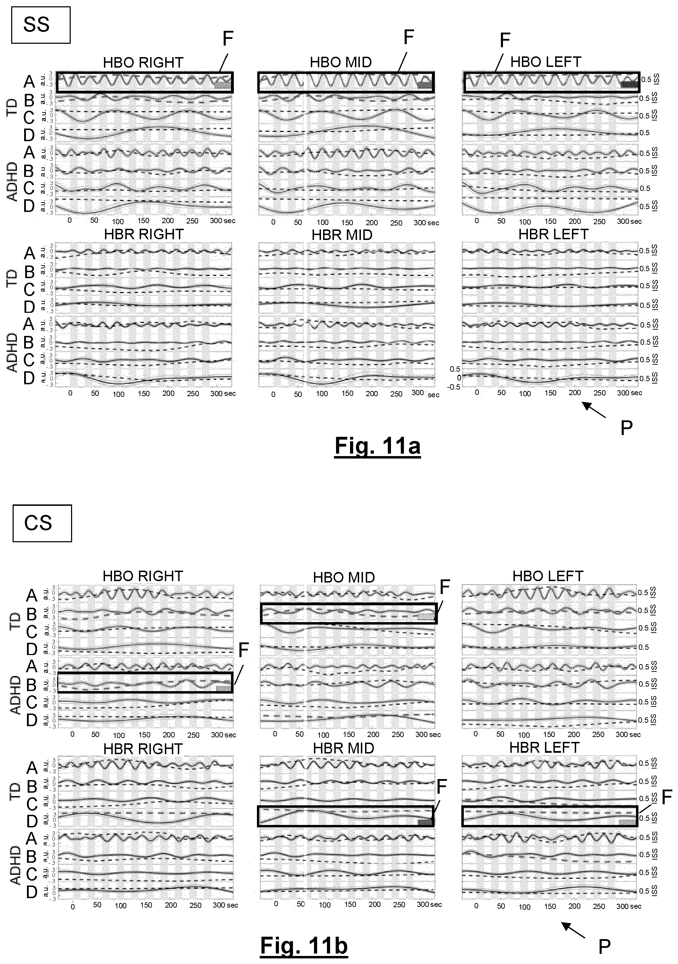

Fig. 11a shows the reference time-series patterns obtained in each sub-band for SS data and Fig. 11b those corresponding to CS data. A rich variety of patterns can be seen, some similar across groups and others clearly different. Thus, for example, TD group exhibits rhythmic fluctuations in the A sub-band of SS-HbO, which are very consistent across participants as reflected by the high ISS (dashed traces); in contrast, ADHD group shows greater inter-subject variability. Another example is visible in CS-HbR-D, were TD group shows a consistent pattern of increasing then decreasing, whereas ADHD group does not. It can also be seen that the ADHD group synchronizes CS-HbO-B towards the end of the task, while TD does so visibly earlier. Once again, certain time-series patterns P seem to represent well the average response of their group, while they do not fit the other one.

Table 2 shows the performance achieved by classifiers CL trained with the features, the functional biomarkers as selected time-series patterns, which were selected by filtering, i.e. those with an $F$ p-value < .01.

Table 2. Performance scores achieved by each classification model trained with the subset of filter-selected features for shallow-signals (SS), clean-signals (CS) and SS + CS.

| Signal type | P. metric | SVM | | LR | | LDA | | NB | |
| | | 5-FO | LOO | 5-FO | LOO | 5-FO | LOO | 5-FO | LOO |
| --- | --- | --- | --- | --- | --- | --- | --- | --- | --- |
| SS 5 features | Accuracy | 73,3 | 70 | 80 | 80 | 74,5 | 73,3 | 80,5 | 80 |
| | Sensitivity | 78,7 | 73,3 | 85,7 | 86,7 | 80,7 | 80 | 88,7 | 86,7 |
| | Specificity | 78,7 | 73,3 | 85,7 | 86,7 | 80,7 | 80 | 88,7 | 86,7 |
| CS 10 features | Accuracy | 81,2 | 80 | 83,5 | 80 | 78,7 | 80 | 85,5 | 86,7 |
| | Sensitivity | 82,3 | 80 | 82 | 80 | 81,7 | 80 | 84,7 | 86,7 |
| | Specificity | 82,3 | 80 | 82 | 80 | 81,7 | 80 | 84,7 | 86,7 |
| CS & SS 15 features | Accuracy | 88,7 | 86,7 | 88,2 | 90 | 85,7 | 83,3 | 85,7 | 86,7 |
| | Sensitivity | 91.3 | 93,3 | 91,7 | 93,3 | 82,7 | 73,3 | 84 | 86,7 |
| | Specificity | 86 | 80 | 84,7 | 86,7 | 88,7 | 93,3 | 87,3 | 86,7 |

Note: LOO =leave-one-out;5-FO= 5 folds;SVM =support vector machine;LR = logistic regression;LDA =linear discriminant analysis; NB = naive Bayes.

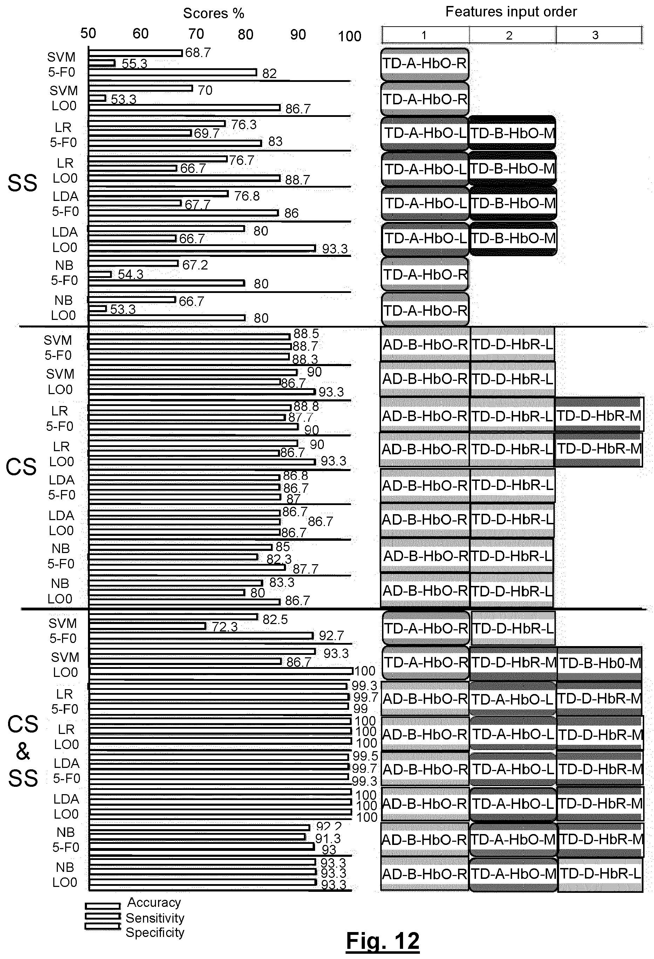

Figure 11a presents shallow-signals (SS) reference time-series patterns P for each frequency sub-band A,B,C,D within each ROI. Upper rows show the HbO patterns for TD and ADHD groups, while lower rows refer to HbR; thin traces depict the time-series pattern's SEM. Dashed lines show the inter-subject synchronization (ISS) time-course in each frequency sub-band A,B,C,D (scale on the right axis); in magenta color those corresponding to time-series patterns with an F p-value < .01. The boxes identify the time-series patterns P chosen by the wrappers as functional biomarkers F (see Fig. 12). Gray shaded rectangles indicate each of the 15 seconds of mental math during stimulation S task. Fig. 11b presents clean-signals (CS) reference time-series patterns P with a description of the drawn elements being the same as in Fig. 11a.

Fig. 12 demonstrates how performance was greatly improved by using the wrapper for feature selection. Note that, in all cases, the classification models were very parsimonious and no more than three features were used. When compared separately, wrapper-selected CS features perform better that SS overall. Regarding the input order, for SS the first-in feature always belong to TD group, A sub-band, HbO, left- or right-ROI depending on the classifier ("TD-A-HbO-L" or "TD-A-HbO-R" in Fig. 9). It should be noted, regarding CS, that all the classifiers agree on the first two features "AD-B-HbO-R" and "TD-D-HbR-L" (see Fig. 12). Once again, the best results were obtained with SS + CS. In fact, LR and LDA scored over 99% on all three metrics for both cross-validation schemes, which is really high performance. NB scored lower, from 91.3% to 93.3% overall while SVM was the weakest classifier when all metrics are considered.

Fig. 12 presents performance scores achieved by each classification model using the subset of wrapper-selected features for shallow-signals (SS), clean-signals (CS) and SS + CS. Rectangles at the right identify the chosen features and their input order. Each specific feature has a different color and its label is the combination "Group-Band-Chromophore-ROI", being TD = TD group, AD = ADHD, L = left, M = mid and R = right.

When evaluating SS+CS, the wrapper selected the same features for LR and LDA in both cross-validation cases. Noteworthy, the first-in feature was "AD-B-HbO-R", which was also the first for CS. The second one was "TD-A-HbO-L", the first for SS. Finally, "TD-D-HbR-M" from CS completed the set. Looking at the wrapper history, we observed that "AD-B-HbO-R" alone achieves about 80% of the accuracy, sensitivity and specificity, which is not surprising since it has the highest F (19.6, p-val < .0001). The addition of "TD-A-HbO-L" improves the scores up to 90% and with "TD-D-HbR-M" they approach 100%. Therefore, the most powerful feature comes from the CS-HbO data of ADHD group, specifically from band B in which a prominent ISS peak can be seen (Fig. 11b). TD group provides the next best feature in form of consistent HbO fluctuations at task frequency in the SS data (see the ISS peak in sub-band A). Finally, a very-slow CS-HbR component of TD group optimizes the classification (see the peak in D). The time-series patterns that generated these features are respectively identified by green, red and blue boxes in Figs. 11a and 11b. Noteworthy, NB also shared the first feature while the second and third come from the same group, sub-band and chromophore but from an adjacent ROI.

Table 3 summarizes the results obtained by wrappers with SS+CS features. In all cases (except for SVM in sensitivity with 5-fold) permutation testing indicated significance at $p < .001$. LR and LDA showed the highest scores in all metrics and also the narrowest CIs. Although highly significant, NB showed lower performance and larger ICs, whereas SVM performed the worst.

Table 3. Performance scores achieved by each classification model using the best subset of wrapper-selected features obtained from shallow- (SS) plus clean-signals (CS). Statistical significance is indicated by p-values and performance 95% Cls are represented within square brackets.

| Signal | P. metric | SVM | | LR | | LDA | | NB | |
|---|---|---|---|---|---|---|---|---|---|
| | | 5-FO | LOO | 5-FO | LOO | 5-FO | LOO | 5-FO | LOO |
| CS & SS | Accuracy | 82.5 $p < .001$ [68.2, 95.7] | 93.3 $p < .001$ [80.0, 100] | 99.3 $p < .001$ [95.8. 100] | 100 $p < .001$ [96.7, 100] | 99.5 $p < .001$ [96.7, 100] | 100 $p < .001$ [96 7, 100] | 92.2 $p < .001$ [80.3, 98.0] | 93.3 $p < .001$ [83.3, 100] |
| | Sensitivity | 72.3 $p = 046$ [56.0. 73.3] | 86.7 $p < 001$ [66.7, 100] | 99.7 $p < .001$ [93.3, 100] | 100 $p < .001$ [93.3, 100] | 99.7 $p < .001$ [92.0, 1001] | 100 $p < .001$ [93.3, 100] | 91.3 $p < 001$ [73.3, 99.7] | 93.3 $p < .001$ 180.0, 100] |
| | Specificity | 92.7 $p < .001$ [78.2, 100] | 100 $p < .001$ [86.7, 100] | 99.0 $p < .001$ [91.8, 100] | 100 $p < .001$ [93.3, 100] | 99.3 $p < .001$ [90.4. 100] | 100 $p < 001$ [93.3, 100] | 93.0 $p < 001$ (80, 100] | 93.3 $p < .001$ [80.0, 100] |
| | Features | 2 | 3 | 3 | 3 | 3 | 3 | 3 | 3 |

Note: LOO = leave-one-out;5-FO = 5-fold;SVM = support vector machine;LR = logistic regression; LDA= linear discriminant analysis; NB=naïve Bayes.

Noteworthy, when the full feature set (48 SS + 48 CS) was used to feed the wrappers for LR and LDA, we got exactly the same feature sub-set for both cross-validation schemes and, hence, the same scores and statistics. Therefore, feature pre-selection did not lead to more optimistic solutions with inflated performance, albeit it did reduce computational cost.

Surprisingly, SVM performed the worst in the SS+CS case, particularly with the 5-fold cross-validation where it achieved the lowest scores and also the widest CIs. In contrast, the other classifiers were not affected by the cross-validation, reaching similar scores and CIs with both schemes (Table 3). Most likely, SVM performance degraded due to lack of proper regularization, leading to insufficient penalty for misclassification. As mentioned, one of the goals was to avoid any hyperparameter tuning and therefore we preferred to rule out SVM in the context of the present study.

As seen from Fig. 12 and Table 3, LR and LDA won in the classification task when trained with SS+CS features. They both agreed on the same wrapper solution, which was exactly the same for the features preselected by filtering and for the full set. Also, they achieved comparable scores (> 99%) in all metrics (significant at p < .001, but now using permutation tests to define the null hypothesis). In addition, the 95% CIs were similarly narrow, although skewed due to scores being close to the 100% ceiling. Cross-validation is known to tend towards narrower confidence bounds as accuracy approaches 100% but increasingly wide and asymmetric as sample size decreases, which can lead to under-estimate prediction errors and specially with LOO cross-validation. Despite having only 30 samples, we found that the lower limit of the CI for accuracy was never < 95.8% and was less than 5% away from the mean in all cases, a deviation below the overall 15% expected for a binary classification with this number of samples. The results suggest that the few selected features meet the statistical assumptions required by LR and LDA to make observations highly separable into 2-classes. The rest of this discussion focuses on LR and LDA and their three wrapper-selected shared features.

LOO and k-fold are the most commonly adopted cross-validation methods in ADHD studies; plus hold-out which seems more appropriate for large datasets. Some researchers have suggested that LOO may be more useful in a diagnostic scenario, whereas others recommend k-fold or repeated random splits for more stable estimates. In either case, it is known that cross-validation is compromised by small sample sizes, particularly if there are many predictors, which tend to overestimate predictive accuracy to a variable degree depending on the particularities of the study. In light of this, we tested LOO and 5-fold expecting differences in performance, with LOO showing more optimistic scores and larger confidence bounds. However, we found that both gave very similar results with LR and LDA (Table 3), suggesting that the three chosen features are good enough predictors to yield stable cross-validation measures regardless of method.

**Claims**

1. Computer implemented method for selecting functional biomarkers (F) to identify a target condition (ADHD) in a subject, the method comprising the steps of:

   a. obtaining groups of equivalent physiological signals (1) generated during a stimulation (S) , comprising a first group (3a) of physiological signals (1) from subjects (2) presenting the target condition (ADHD), and a second group (3b) of physiological signals (1) from subjects (2) of control condition (TD), not presenting the target condition (ADHD);
   b. identifying frequency sub-bands (A,B,C,D) for each group (3a,3b) of physiological signals corresponding to frequencies that show higher group synchronization;
   c. obtaining per each identified frequency sub-band (A,B,C,D) a corresponding time-series pattern (P);
   d. selecting the one or more time-series patterns (P) having similarity values (d) to the physiological signals (1) of each group (3a,3b) that differentiate the groups of physiological signals (1) as functional biomarkers (F) to identify the target condition (ADHD);
   wherein the step of identifying frequency sub-band (A,B,C,D) comprises, per each group (3a,3b):

      b1. obtaining a time-frequency transform (T) of each physiological signal (1);
      b2. averaging the time-frequency transforms (T);
      b3. extracting the local-maxima (Lmax) of the averaged time-frequency transforms (T) ; and
      b4. identifying the frequency sub-bands (A,B,C,D) for the group as frequency ranges around the local-maxima (Lmax);

   and wherein the step of obtaining per each identified frequency sub-band (A,B,C,D) a corresponding time-series pattern (P), per each group (3a,3b) comprises:

      c1. computing an inverse time-frequency transform (IT) from the calculated time-frequency transform (T) of the physiological signals (1) per each frequency sub-band (A,B,C,D) to obtain a corresponding time-series (t)

16

for each member of the group (3a,3b); and

c2. generating a time-series pattern (P) of the group by averaging the obtained time-series (t) for each member of the group.

2. Computer implemented method according to the preceding claim, **characterized in that** the stimulation (S) is a periodical task having a task frequency, the frequency sub-band (A,B,C,D) including the task frequency.

3. Computer implemented method according to any of the preceding claims, **characterized in that** the physiological signal (1) is one of a cerebral signal, such as a NIRS signal, a fMRI-BOLD signal, or an EEG signal, or an hemodynamic signal such as a blood pressure or flow signal.

4. Computer implemented method according to any of the preceding claims, **characterized in that** the physiological signal (1) is a cerebral signal, obtained in a same region of the brain, in response to relative or absolute changes in the concentration of a hemoglobin chromophore.

5. Computer implemented method according to any of the preceding claims, **characterized in that** the time-frequency transform (T) is a continuous wavelet transform (CWT) .

6. Computer implemented method according to any of the preceding claims, **characterized in that** selecting the one or more time-series patterns (P) that better differentiate the groups of physiological signals (1) comprises calculating time-series similarity values (d) between each candidate time-series pattern (P) and the time-series (t) of each member of the group (3a,3b), and selecting the time-series patterns (P) with similarity values that better differentiate the groups as functional biomarkers (F).

7. Computer implemented method according to any of the preceding claims, **characterized in that** the step of identifying frequency sub-bands (A,B,C,D) comprises calculating an inter-subject synchronization (ISS) measure.

8. Computer implemented method according to the preceding claim **characterized in that** the inter-subject synchronization (ISS) measure comprises a phase synchronization measure.

9. Computer implemented method according to the preceding claim **characterized in that** the inter-subject synchronization (ISS) measure comprises a combination of a phase and magnitude synchronization measure.

10. Computer implemented method according to any of the preceding claims, **characterized in that** time-series patterns (P) are selected based on statistical contrast at a predefined significance level.

11. Computer implemented method according to any of the preceding claims, **characterized in that** it further comprises training a classifier (CL) for identifying the target condition (ADHD) using the similarity values (d) to the selected functional biomarkers (F).

12. A computer program including instructions that when executed cause a machine to perform the method of any of the claims 1 to 11.

**Patentansprüche**

1. Computerimplementiertes Verfahren zur Auswahl funktioneller Biomarker (F) zur Identifizierung eines Zielzustands (ADHD) in einem Individuum, wobei das Verfahren die folgenden Schritte umfasst:

a. das Erhalten von Gruppen von äquivalenten physiologischen Signalen (1), welche während einer Stimulation (S) erzeugt werden, welche eine erste Gruppe (3a) von physiologischen Signalen (1) aus Individuen (2), welche den Zielzustand (ADHD) aufweisen, und eine zweite Gruppe (3b) von physiologischen Signalen (1) aus Individuen (2) des Kontrollzustands (TD), welche den Zielzustand (ADHD) nicht aufweisen, umfassen;

b. das Identifizieren von Frequenzteilbändern (A, B, C, D) für jede Gruppe (3a, 3b) von physiologischen Signalen, welche den Frequenzen entsprechen, welche eine höhere Gruppensynchronisation zeigen;

c. das Erhalten, pro jedes identifizierte Frequenzteilband (A, B, C, D), eines entsprechenden Zeitreihenmusters (P);

d. das Auswählen des einen oder der mehreren Zeitreihenmuster (P) aufweisend Ähnlichkeitswerte (d) zu den physiologischen Signalen (1) jeder Gruppe (3a, 3b), welche die Gruppen von physiologischen Signalen (1) als funktionelle Biomarker (F) unterscheiden, um den Zielzustand (ADHD) zu identifizieren;

wobei der Schritt des Identifizierens des Frequenzteilbands (A, B, C, D), pro jede Gruppe (3a, 3b) Folgendes umfasst:

b1. das Erhalten einer Zeit-Frequenz-Transformierte (T) von jedem physiologischen Signal (1);
b2. das Durchschnittsermitteln der Zeit-Frequenz-Transformierten (T);
b3. das Gewinnen der lokalen Maxima (Lmax) der durchschnittlichen Zeit-Frequenz-Transformierten (T); und
b4. das Identifizieren der Frequenzteilbänder (A, B, C, D) für die Gruppe als Frequenzbereiche um die lokalen Maxima (Lmax) herum;

und wobei der Schritt des Erhaltens, pro jedes identifizierte Frequenzteilband (A, B, C, D), eines entsprechenden Zeitreihenmusters (P), pro jede Gruppe (3a, 3b) Folgendes umfasst:

c1. das Errechnen einer inversen Zeit-Frequenz-Transformierte (IT) aus der berechneten Zeit-Frequenz-Transformierte (T) der physiologischen Signale (1) pro jedes Frequenzteilband (A, B, C, D), um eine entsprechende Zeitreihe (t) für jedes Mitglied der Gruppe (3a, 3b) zu erhalten; und
c2. das Erzeugen eines Zeitreihenmusters (P) der Gruppe, indem für die erhaltene Zeitreihe (t) für jedes Mitglied der Gruppe der Durchschnitt ermittelt wird.

2. Computerimplementiertes Verfahren nach dem vorhergehenden Anspruch, **dadurch gekennzeichnet, dass** die Stimulation (S) eine periodische Aufgabe ist, welche eine Aufgabenfrequenz aufweist, wobei das Frequenzteilband (A, B, C, D) die Aufgabenfrequenz beinhaltet.

3. Computerimplementiertes Verfahren nach einem der vorhergehenden Ansprüche, **dadurch gekennzeichnet, dass** das physiologische Signal (1) eins aus einem Gehirnsignal, wie einem NIRS-Signal, einem fMRI-BOLD-Signal oder einem EEG-Signal, oder einem hämodynamischen Signal, wie einem Blutdruck- oder Blutflusssignal, ist.

4. Computerimplementiertes Verfahren nach einem der vorhergehenden Ansprüche, **dadurch gekennzeichnet, dass** das physiologische Signal (1) ein Gehirnsignal ist, erhalten in einem gleichen Bereich des Gehirns, als Antwort auf relative oder absolute Änderungen bei der Konzentration eines Hämoglobin-Chromophors.

5. Computerimplementiertes Verfahren nach einem der vorhergehenden Ansprüche, **dadurch gekennzeichnet, dass** die Zeit-Frequenz-Transformierte (T) eine kontinuierliche Wavelet-Transformierte (CWT) ist.

6. Computerimplementiertes Verfahren nach dem vorhergehenden Anspruch, **dadurch gekennzeichnet, dass** das Auswählen des einen oder der mehreren Zeitreihenmuster (P), welche die Gruppen von physiologischen Signalen (1) besser unterscheiden, das Berechnen von Zeitreihen-Ähnlichkeitswerten (d) zwischen jedem Kandidat-Zeitreihenmuster (P) und der Zeitreihe (t) jedes Mietglieds der Gruppe (3a, 3b) und das Auswählen der Zeitreihenmuster (P) mit Ähnlichkeitswerten, welche die Gruppen als funktionelle Biomarker (F) besser unterscheiden, umfasst.

7. Computerimplementiertes Verfahren nach einem der vorhergehenden Ansprüche, **dadurch gekennzeichnet, dass** der Schritt des Identifizierens der Frequenzteilbänder (A, B, C, D) das Berechnen einer interindividuellen Synchronisations (ISS)-Messung umfasst.

8. Computerimplementiertes Verfahren nach dem vorhergehenden Anspruch, **dadurch gekennzeichnet, dass** die interindividuelle Synchronisations (ISS)-Messung eine Phasensynchronisationsmessung umfasst.

9. Computerimplementiertes Verfahren nach dem vorhergehenden Anspruch, **dadurch gekennzeichnet, dass** die interindividuelle Synchronisations (ISS)-Messung eine Kombination einer Phasen- und Magnitudensynchronisationsmessung umfasst.

10. Computerimplementiertes Verfahren nach einem der vorhergehenden Ansprüche, **dadurch gekennzeichnet, dass** Zeitreihenmuster (P) basierend auf dem statistischen Kontrast bei einem vordefinierten Signifikanzniveau ausgewählt werden.

11. Computerimplementiertes Verfahren nach einem der vorhergehenden Ansprüche, **dadurch gekennzeichnet, dass** es zusätzlich das Trainieren eines Klassierers (CL) zur Identifizierung des Zielzustands (ADHD) unter Verwendung der Ähnlichkeitswerte (d) zu den funktionellen Biomarkern (F) umfasst.

12. Computerprogramm beinhaltend Anweisungen, welche, wenn sie ausgeführt werden, eine Maschine dazu bringen, das Verfahren nach einem der Ansprüche 1 bis 11 durchzuführen.

**Revendications**

1. Procédé mis en œuvre par ordinateur pour sélectionner des biomarqueurs fonctionnels (F) pour identifier un état cible (TDAH) chez un sujet, le procédé comprenant les étapes consistant à:

   a. obtenir des groupes de signaux physiologiques équivalents (1) générés pendant une stimulation (S), comprenant un premier groupe (3a) de signaux physiologiques (1) à partir de sujets (2) présentant l'état cible (TDAH), et un deuxième groupe (3b) de signaux physiologiques (1) à partir de sujets (2) d'état témoin (TD), qui ne présentent pas l'état cible (TDAH);
   b. identifier des sous-bandes de fréquence (A, B, C, D) pour chaque groupe (3a, 3b) de signaux physiologiques correspondant à des fréquences qui présentent une synchronisation de groupe supérieure;
   c. obtenir, pour chaque sous-bande de fréquence (A, B, C, D) identifiée, un tracé de série temporelle (P) correspondant;
   d. sélectionner l'un ou plusieurs tracés de série temporelle (P) ayant des valeurs de similarité (d) par rapport aux signaux physiologiques (1) de chaque groupe (3a, 3b) qui distinguent les groupes de signaux physiologiques (1) en tant que biomarqueurs fonctionnels (F) pour identifier l'état cible (TDAH);
   dans lequel l'étape d'identifier des sous-bandes de fréquence (A, B, C, D) comprend, pour chaque groupe (3a, 3b):

      b1. obtenir une transformée de temps-fréquence (T) de chaque signal physiologique (1);
      b2. calculer la moyenne des transformées de temps-fréquence (T);
      b3. extraire les maxima locaux (Lmax) des transformées de temps-fréquence moyennées (T); et
      b4. identifier les sous-bandes de fréquence (A, B, C, D) pour le groupe en tant qu'intervalles de fréquence autour des maxima locaux (Lmax);

   et dans lequel l'étape d'obtenir, pour chaque sous-bande de fréquence (A, B, C, D) identifiée, un tracé de série temporelle (P) correspondant, pour chaque groupe (3a, 3b), comprend:

      c1. calculer une transformée inverse de temps-fréquence (IT) à partir de la transformée de temps-fréquence (T) calculée des signaux physiologiques (1) pour chaque sous-bande de fréquence (A, B, C, D) pour obtenir une série temporelle (t) correspondante pour chaque membre du groupe (3a, 3b); et
      c2. générer un tracé de série temporelle (P) du groupe en calculant la moyenne de la série temporelle (t) obtenue pour chaque membre du groupe.

2. Procédé mis en œuvre par ordinateur selon la revendication précédente, **caractérisé en ce que** la stimulation (S) est une tâche périodique ayant une fréquence de tâche, la sous-bande de fréquence (A, B, C, D) comportant la fréquence de tâche.

3. Procédé mis en œuvre par ordinateur selon l'une quelconque des revendications précédentes, **caractérisé en ce que** le signal physiologique (1) est l'un parmi un signal cérébral, tel qu'un signal de NIRS, un signal de fMRI-BOLD, ou un signal d'EEG, ou un signal hémodynamique tel qu'un signal de tension artérielle ou débit sanguin.

4. Procédé mis en œuvre par ordinateur selon l'une quelconque des revendications précédentes, **caractérisé en ce que** le signal physiologique (1) est un signal cérébral, obtenu dans une même région du cerveau, en réponse à des changements relatifs ou absolus dans la concentration d'un chromophore de l'hémoglobine.

5. Procédé mis en œuvre par ordinateur selon l'une quelconque des revendications précédentes, **caractérisé en ce que** la transformée de temps-fréquence (T) est une transformée en ondelettes continue (CWT).

6. Procédé mis en œuvre par ordinateur selon l'une quelconque des revendications précédentes, **caractérisé en ce que** la sélection de l'un ou plusieurs tracés de série temporelle (P) qui distinguent mieux les groupes de signaux physiologiques (1) comprend le calcul de valeurs de similarité de série temporelle (d) entre chaque tracé de série temporelle (P) candidat et la série temporelle (t) de chaque membre du groupe (3a, 3b), et la sélection des tracés de série temporelle (P) avec des valeurs de similarité qui distinguent mieux les groupes en tant que biomarqueurs fonctionnels (F).

**7.** Procédé mis en œuvre par ordinateur selon l'une quelconque des revendications précédentes, **caractérisé en ce que** l'étape d'identifier des sous-bandes de fréquence (A, B, C, D) comprend le calcul d'une mesure de synchronisation inter-sujets (ISS).

**8.** Procédé mis en œuvre par ordinateur selon la revendication précédente, **caractérisé en ce que** la mesure de synchronisation inter-sujets (ISS) comprend une mesure de synchronisation de phase.

**9.** Procédé mis en œuvre par ordinateur selon la revendication précédente, **caractérisé en ce que** la mesure de synchronisation inter-sujets (ISS) comprend une combinaison d'une mesure de synchronisation de phase et d'amplitude.

**10.** Procédé mis en œuvre par ordinateur selon l'une quelconque des revendications précédentes, **caractérisé en ce que** des tracés de série temporelle (P) sont sélectionnés sur la base d'un contraste statistique à un niveau de signification prédéfini.

**11.** Procédé mis en œuvre par ordinateur selon l'une quelconque des revendications précédentes, **caractérisé en ce qu'**il comprend en outre l'entrainement d'un classificateur (CL) pour identifier l'état cible (TDAH) en utilisant les valeurs de similarité (d) par rapport aux biomarqueurs fonctionnels (F) sélectionnés.

**12.** Programme informatique comportant des instructions qui, lorsqu'elles sont exécutées, amènent une machine à réaliser le procédé selon l'une quelconque des revendications 1 à 11.

**Fig. 1**

3a

1

3b

1

ADHD

TD

d

CL

ADHD

TD

F

**Fig. 2**

1

d

CL

ADHD

TD

F

**Fig. 3**

| 300 sec | Calculation 15 sec | Pause 15 sec | Calculation 15 sec | Pause 15 sec | | Calculation 15 sec | Pause 15 sec | 300 sec |
|---|---|---|---|---|---|---|---|---|
| Relax | 112 + 5 | ? Relax | 105 + 4 | ? Relax | | 123 + 8 | ? Relax | Relax |
| BASELINE | Trial 1 | | Trial 2 | | | Trial 10 | | RECOVERY |

**Fig. 4**

**Fig. 5**

**Fig. 6**

**Fig. 7a**

**Fig. 7b**

**Fig. 8**

**Fig. 9a**

**Fig. 9b**

Fig. 10

**Fig. 11a**

**Fig. 11b**

**Fig. 12**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MONDEN et al.** *Individual classification of ADHD children by right prefrontal hemodynamic responses during a go/no-go task as assessed by fNIRS*, 2015
- **CRIPPA et al.** *The utility of a computerized algorithm based on a multi-domain profile of measures for the diagnosis of attention deficit/hyperactivity disorder*, 2017

- **GU et al.** *Identifying ADHD children using hemodynamic responses during a working memory task measured by functional near-infrared spectroscopy*, 2018
- **GHORBANIAN P. et al.** Continuous Wavelet Transform EEG Features of Alzheimer's Disease. *ASME 2012 5TH ANNUAL DYNAMIC SYSTEMS AND CONTROL CONFERENCE JOINT WITH THE JSME 2012 11TH MOTION AND VIBRATION CONFERENCE*, 13 January 2015, 567-572